(19) Europäisches Patentamt
European Patent Office
Office européen des brevets

(11) Publication number: **0 038 661**
**B1**

(12) **EUROPEAN PATENT SPECIFICATION**

(45) Date of publication of patent specification: **28.12.83**

(21) Application number: **81301622.7**

(22) Date of filing: **14.04.81**

(51) Int. Cl.[3]: **C 07 D 205/08,**
C 07 D 205/12 //C07F15/02,
C07D405/04

(54) **Process for the preparation of beta-lactams.**

(30) Priority: **21.04.80 GB 8013001**

(43) Date of publication of application:
**28.10.81 Bulletin 81/43**

(45) Publication of the grant of the patent:
**28.12.83 Bulletin 83/52**

(84) Designated Contracting States:
**CH DE FR GB IT LI NL**

(56) References cited:

**JOURNAL OF THE AMERICAN CHEMICAL SOCIETY, vol. 99, no. 8, April 13, 1977, page 2823-4 P.K. WONG et al.: "A New Synthesis of beta-Lactams"**

**JOURNAL OF THE CHEMICAL SOCIETY CHEMICAL COMMUNICATIONS, no. 17, September 7, 1977, pages 581-582 G.D. ANNIS et al.: "Formation of Lactones from Dienes via Iron Carbonyl Complexes"**

(73) Proprietor: **BEECHAM GROUP PLC**
**Beecham House Great West Road**
**Brentford Middlesex (GB)**

(72) Inventor: **Ley, Steven Victor**
**42 The Ruffetts Ballards Farm**
**Sanderstead Surrey (GB)**
Inventor: **Hebblethwaite, Elizabeth Mary**
**10 Gordon Terrace Idle**
**Bradford Yorkshire (GB)**

(74) Representative: **Hesketh, Alan, Dr. et al,**
**European Patent Attorney Beecham Pharmaceuticals Great Burgh Yew Tree Bottom Road**
**Epsom, Surrey, KT18 5XQ (GB)**

**EP 0 038 661 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Process for the preparation of $\beta$-lactams

This invention relates to a chemical process for the preparation of $\beta$-lactams, which are themselves useful as intermediates in the production of antibacterial compounds.

J. Chem. Soc. Chem. Comm., 1977,851 and J. Organometallic Chem., 1979, *182*, C11 disclose a process for the preparation of $\beta$- and $\delta$-lactones, which process comprises oxidation of a compound of formula I:

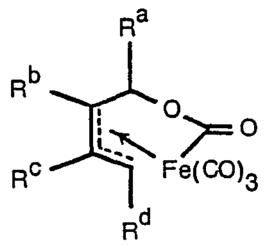

(I)

wherein $R^a$ and $R^d$ are hydrogen and $R^b$ and $R^c$ are methyl, $R^b$ and $R^c$ are hydrogen and $R^a$ and $R^d$ together represent $-(CH_2)_2-$, or $R^a$ and $R^b$ together represent $-(CH_2)_3-$ and $R^c$ and $R^d$ are hydrogen; with a cerium IV salt.

J. Amer. Chem. Soc., 1977, *99*, no 8 April 13 page 2823—4 discloses a new synthesis of $\beta$-Lactams using $\eta^5$-cyclopentadienyliron (olefin)-amine adducts.

It has now been found that high yields of $\beta$-lactams may be obtained by oxidation of tricarbonyl-iron-$\pi$-allyl-$\sigma$-lactam complexes.

Accordingly the present invention provides a process for the preparation of a lactam of formula (II):

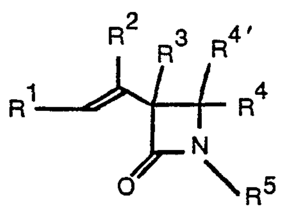

(II)

wherein $R^1$, $R^2$ and $R^3$ may be the same or different and each represents hydrogen or $C_{1-6}$ alkyl, or $R^1$ and $R^2$ may together complete a carbocyclic ring; $R^4$ is hydrogen, $-CH_2-CO_2R^p$ where $R^p$ is a carboxy-protecting group, $-CH_2-CH_2-OH$ or a protected derivative thereof, $-CH_2-CH_2-CHO$ or a protected derivative thereof, or $R^3$ and $R^4$ together complete a carbocyclic ring; $R^{4'}$ is hydrogen or $C_{1-6}$ alkyl; and $R^5$ is hydrogen, an organic radical wherein any reactive groups may be protected or a subgroup of formula-$NR^\alpha R^\beta$ wherein $R^\alpha$ and $R^\beta$ are the same or different and each is hydrogen or an organic radical, or a subgroup of $OR^\delta$ wherein $R^\delta$ is a hydroxyl protecting group, which process comprises treatment of an iron complex of formula (III):

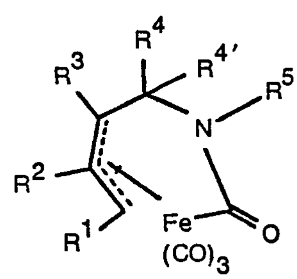

(III)

wherein $R^1$, $R^2$, $R^3$, $R^4$, $R^{4'}$ and $R^5$ are as defined with respect to a formula (II), with an oxidising agent.

Compounds of formula (II) contain at least one chiral centre *viz* the carbon atom marked 3 below. When $R^4$ and $R^{4'}$ are not the same the compounds have a second chiral centre carbon 4.

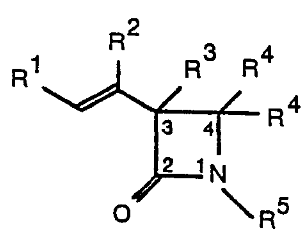

Thus when we refer generally to compounds of formula (II) herein, we mean mixtures of stereochemical isomers as well as pure isomers.

This cyclisation may be carried out in a range of organic solvents subject to the solubility of the iron complex (III) and the oxidizing agent. It is convenient to use a polar solvent, preferably a water-miscible solvent such as, for example, tetrahydrofuran, acetone, dimethylformamide, dimethylsulphoxide, hexamethylphosphoramide, acetonitrile, dimethoxyethane, dioxan or an alcohol such as methanol, ethanol, propanol or butanol.

Suitable solvents include acetonitrile or ethanol. Preferably the solvent is ethanol.

The oxidising agent used in the above process should have a finite solubility in the reaction solvent. Suitable oxidising agents include iron III salts, silver I salts, and cerium IV salts. A preferred oxidising agent is a cerium IV salt, in particular cerium IV ammonium nitrate.

The initial stage of the process is generally carried out at moderate to low temperatures such as, for example −50° to +30°C, and the reaction mixture allowed to equilibrate to room temperature during the course of the reaction. The period for which the reaction is allowed to proceed depends upon the particular starting materials employed. The course of the reaction may be followed by conventional methods such as thin layer chromatography and terminated when an optimum quantity of product is present in the reaction mixture.

Suitably the group $R^5$ is hydrogen, an amino protecting group, such as for example p-nitrobenzyl, or a sub-group of formula:

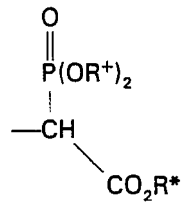

wherein $R^+$ is $C_{1-6}$ alkyl and $R^*$ is an organic group such that $—CO_2R^*$ is an ester group.

Suitable groups $R^+$ include methyl, ethyl, and *n*- and *iso*-propyl.

Certain compounds within formula (III) are disclosed in Tetrahedron, 1976; *32*, 2123, Tetrahedron, 1974, 30, 839, and Chemische Berichte, 1979, *112*, 3644. Compounds of formula (III) may be prepared by reaction of a metal complex of formula (IV):

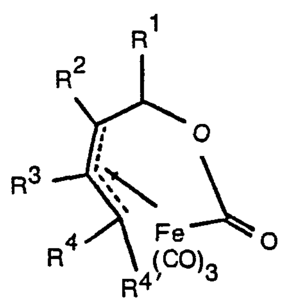

(IV)

wherein $R^1$, $R^2$, $R^3$, $R^4$ and $R^{4'}$ are as defined with respect to formula (II) with a compound of formula (V):

$$R^5—NH_2 \qquad (V)$$

wherein $R^5$ is as defined with respect to formula (II) in the presence of a Lewis acid. Suitable Lewis acids include zinc chloride. An excess of the compound of formula (V) is used in the reaction to ensure a reasonable extent of reaction; preferably a 5 to 10-fold excess is used. The groups $R^1$, $R^2$, $R^3$, $R^4$, $R^{4'}$ and $R^5$ are chosen according to the $\beta$-lactam antibiotic to be synthesised using the compound of formula (II) as starting material.

Suitably $R^5NH_2$ is ammonia, hydrazine, hydroxylamine, a primary amine, N-monosubstituted hydrazine, N,N-disubstituted hydrazine or O-substituted hydroxylamine.

Compounds of formula (II) are useful intermediates in the synthesis of $\beta$-lactam antibiotics.

In a preferred embodiment of the present invention the groups $R^4$ and $R^5$ are selected to facilitate ring closure to produce carbapenem derivatives of formula (VI):

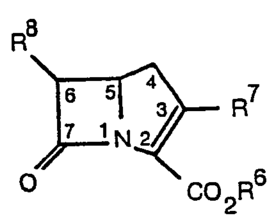

(VI)

wherein $R^6$ is a carboxyl blocking group, $R^7$ is hydrogen, $C_{1-6}$ alkyl or a group —S—$R^9$, wherein $R^9$ is an organic radical, and $R^8$ is ethyl, ethenyl or a group of sub-formula (a):

$$—CH(CH_3)—OR^{10} \qquad (a)$$

wherein $R^{10}$ is hydrogen or an acyl group or a group of formula $R^{11}O_3S$— wherein $R^{11}$ is a salting ion or a methyl or ethyl group.

Formula (VI) includes compounds with antibacterial activity *per se*; however further antibacterially active compounds may be produced by removal of the carboxyl blocking group $R^6$ to produce the free carboxyl group.

A conventional reaction sequence leading to certain compounds of formula (VI), starting from compound (VII) is illustrated in scheme I.

Compound (VII) is an example of a monocyclic β-lactam (II) produced by the process of the present invention. It will be seen from the scheme that the process of the present invention may be used for the preparation of intermediates for pharmaceutically useful compounds.

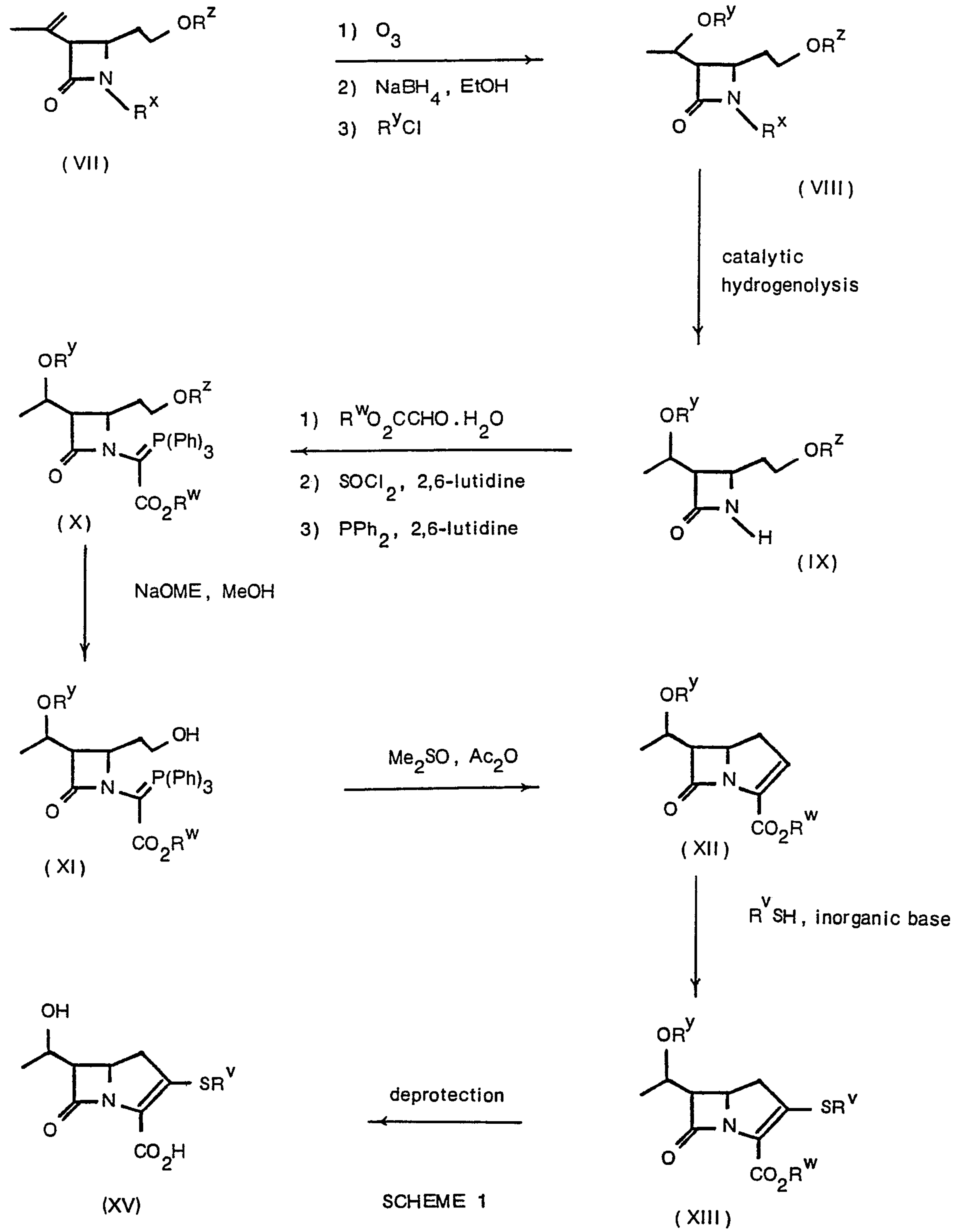


(VII) (VIII) (IX) (X) (XI) (XII) (XIII) (XV)

SCHEME 1

The starting material (VII):

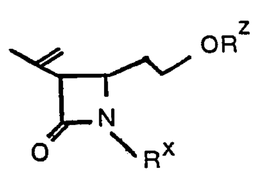

(VII)

wherein $R^z$ is an hydroxyl-protecting group removable by base hydrolysis; and $R^x$ is an amino-protecting

group may be produced by the process of the present invention from the iron complex of formula (XIV):

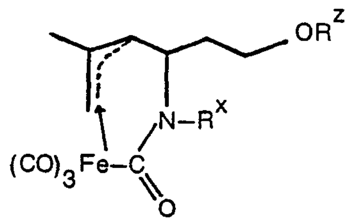

(XIV)

wherein $R^z$ and $R^x$ are as defined hereinbefore. Suitably $R^z$ may be an acetyl group and $R^x$ a p-nitrobenzyl group.

Conversion of compound (VII) to compound (VIII) comprises (1) ozonolysis of the carbon-carbon double bond, (2) reduction of the ozonide produced with sodium borohydride in ethanol to give a free hydroxyl compound, which may then be (3) treated with a compound $R^yCl$ such that $R^y$ is a hydroxyl protecting group.

Compound (IX) may be prepared by catalytic hydrogenolysis of the fully protected compound (VIII).

Compound (X) may be produced from (IX) in three steps, the first being reaction with a suitably protected derivative of glyoxylate hydrate. Suitably $R^w$ may be a p-nitrobenzyl group. The second step comprises treatment with thionyl chloride in the presence of 2,6-lutidine; the third step being treatment with a phosphine such as triphenylphosphine in the presence of 2,6-lutidine.

The compound of formula (X) should then be treated with sodium methoxide in methanol to remove the hydroxyl protecting group $R^z$, giving the compound of formula (XI). The free hydroxyl group in compound (XI) may be cyclised to give the carbapenem derivative (XII) *via* an oxidation step using acetic anhydride in dimethylsulphoxide.

The compound of formula (XIII) is obtained by reaction of the carbapenem derivative (XII) with a thiol of formula $R^vSH$, such that $R^v$ is an organic radical wherein any reactive groups may be protected, in the presence of an inorganic base. Suitably the inorganic base is a carbonate, in particular an alkali metal carbonate such as potassium carbonate. The reaction is conveniently carried out in a polar organic solvent such as dimethylformamide at moderate temperatures i.e. not in excess of 40°C, room temperature being most appropriate.

Compounds of formula (II) wherein $R^5$ is a hydroxyl protected derivative of $-CH_2CH_2OH$ and $R^5$ is a sub group of formula:

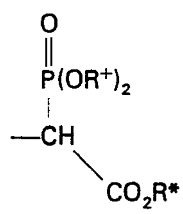

wherein $R^+$ and $R^*$ are as hereinbefore defined, are useful intermediates in a further process for preparing bicyclic β-lactam compounds. A conventional reaction sequence leading to a class of compounds within formula (VI) is illustrated in scheme 2.

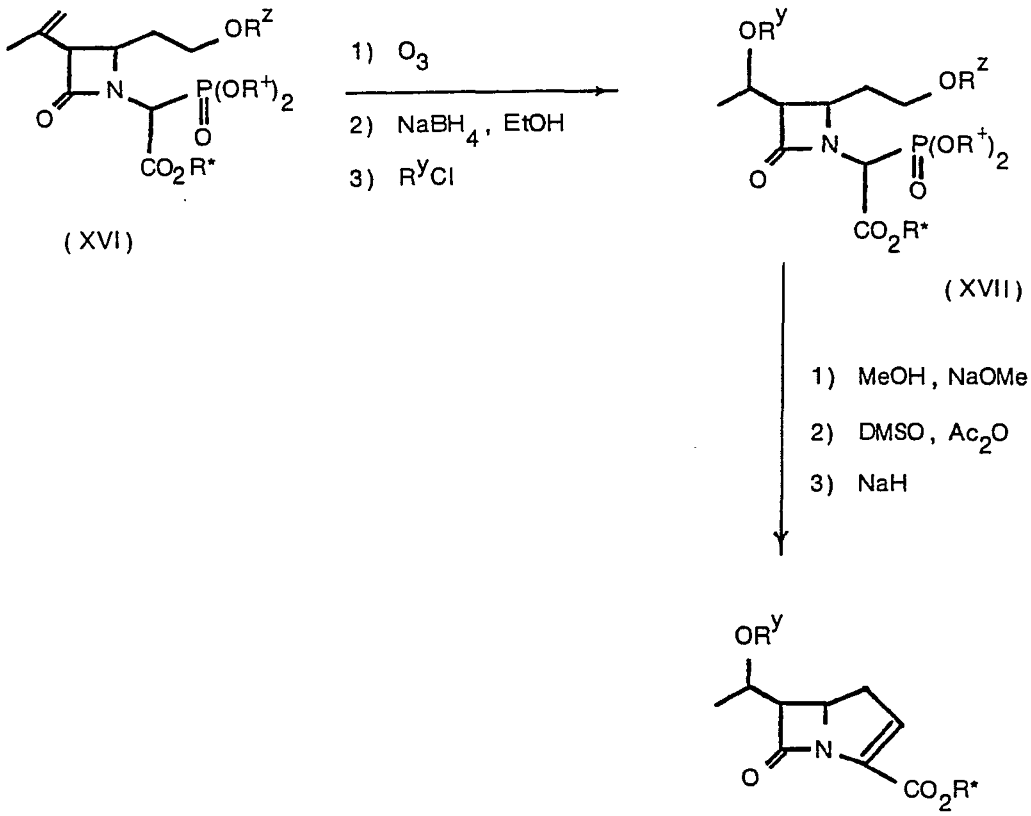


(XVI)

(XVII)

SCHEME 2

(XVIII)

The compounds of the formula (XVIII), wherein $R^z$, $R^+$ and $R^*$ are as described hereinbefore, may be obtained from compound of formula (XVII), wherein $R^z$, $R^y$, $R^+$ and $R^*$ are as described hereinbefore, by a process which comprises (1) removal of the hydroxyl-protecting group $R^z$ by base hydrolysis with sodium methoxide in methanol, (2) treatment with acetic anhydride in dimethylsulphoxide and (3) treatment with sodium hydride.

Compounds of formula (XVII) are prepared in turn from compounds of formula (XVI), wherein $R^z$, $R^+$ and $R^*$ are as described hereinbefore, by a conventional sequence of reactions such as described in Scheme 1 for the conversion of compound (VII) to (VIII).

The following examples illustrate this invention. The following descriptions relate to the preparation of useful starting materials.

M.p.'s were determined with a Kofler hot-stage apparatus. $^1H$ nmr spectra were obtained for solutions in $CDCl_3$ ($Me_4Si$ as internal standard). Petroleum ether refers to the fraction b.p. 40—60°. Solutions were dried over sodium sulphate, and solvents by standard methods. Chromatography was performed on Merck-Kieselgel 60 (0.04—0.063 mm) and (0.063—0.2 mm).

*General Procedure for Tricarbonyl Iron Lactam Complex Preparation*

Benzylamine was added to a solution of the tricarbonyl iron lactone complex and zinc chloride in tetrahydrofuran (THF)/ether or ether. When the reaction was complete (as indicated by thin layer chromatography) the mixture was filtered and the solvent removed under reduced pressure to give the crude product, which was purified by chromatography and subsequent recrystallisation from petroleum ether.

Description 1

Tricarbonyl-(2,3,4-$\eta$-3-buten-2-yl)-1-benzylaminocarbonyl iron (1)

Tricarbonyl-(2,3,4-$\eta$-3-buten-2yl)-1-oxycarbonyl iron (100 mg, 0.42 mmol) with zinc chloride (110 mg, 0.84 mmol) and benzylamine (540 mg, 5 mmol) in THF/ether (1:3, 5 ml) gave tricarbonyl-(2,3,4-$\eta$-3-buten-2-yl)-1-benzylaminocarbonyl iron (1) (117 mg, 81%), m.p. 76—78°C, $\nu_{max.}$ 3080, 2980, 2850, 2070, 2010, 1585, 1570, 1405 and 1165 $cm^{-1}$; $\delta$ 7.35—7.04 (5H, m), 4.84 (1H, ddd J 14, 8 and 8 Hz), 4.40 (1H, m), 4.26 (1H, ABq, J 15 Hz); 4.14 (1H, ABq, J 15 Hz), 3.73 (1H, ddd, J 8, 2 and 2 Hz), 3.46—3.20 (2H, m), and 2.88 (1H, dd, J 12 and 2 Hz) (Found: C, 54.81; H, 3.93; N, 4.25. $C_{15}H_{13}NO_4Fe$ requires C, 55.08; H, 4.01; N, 4.28%.)

Description 2
Tricarbonyl-(2,3,4-η-3-methyl-3-buten-2-yl)-1-benzylaminocarbonyl iron (2)

Tricarbonyl-(2,3,4-η-2-methyl-3-buten-2-yl)-1-oxycarbonyl iron (100 mg, 0.4 mmol) with zinc chloride (107 mg, 0.8 mmol) and benzylamine (510 mg, 4.7 mmol) in THF/ether (1:3, 5 ml) gave tricarbonyl-(2,3,4-η-3-methyl-3-2-yl)-1-benzylaminocarbonyl iron (2) (131 mg, 97%), m.p. 70—72°, $\nu_{max.}$ 3025, 2920, 2850, 2000, 1980, 1590, 1175, 700 and 665 $cm^{-1}$; δ 7.34-.7.06 (5H, m), 4.24 (3H, m), 3.56 (1H, dd J 2 and 2 Hz) 3.33 (1H, ABX, J 13 and 6 Hz) 3.14 (1H, ABX, J 13 and 2 Hz), 2.70 (1H, d, J 2 Hz), and 1.94 (3H, s) (Found: C, 56.04; H, 4.40; N, 4.07. $C_{16}H_{15}NO_4Fe$ requires C, 56.33; H, 4.43; N, 4.12%).

Description 3
Tricarbonyl-(2,3,4-η-2,3-dimethyl-3-buten-2-yl)-1-benzylaminocarbonyl iron (3)

Tricarbonyl-(2,3,4-η-2,3-dimethyl-3-buten-2-yl)-1-oxycarbonyl iron (100 mg, 0.37 mmol) with zinc chloride (100 mg, 0.735 mmol) and benzylamide (483 mg, 4.5 mmol) in ether (6 ml) gave tricarbonyl-(2,3,4-η-2,3-dimethyl-3-buten-2-yl)-1-benzylaminocarbonyl iron (3) (100 mg, 75%), m.p. 98—100°, $\nu_{max.}$ 2900, 2025, 1990, 1570, 1440, 1185 and 705 $cm^{-1}$; δ 7.33—7.08 (5H, m), 4.28 (2H, s), 3.42 (1H, d, J 2 Hz), 3.14 (2H, s), 2.44 (1H, d, J 2 Hz), 2.08 (3H, s), and 1.89 (3H, s) (Found: C, 57.55; H, 4.80; N, 3.90. $C_{17}H_{17}NO_4$ Fe requires C, 57.50; H, 4.80; N, 3.95%).

Description 4
E-tricarbonyl-(2,3,4-η-3-nonen-2-yl)-1-benzylaminocarbonyl iron (4)

A mixture of cis- and trans tricarbonyl-(1,2,3-η-1-nonen-3-yl)-4-oxycarbonyl iron (460 mg, 1.49 mmol) with zinc chloride (200 mg, 1.47 mmol) and benzylamine (750 mg, 7 mmol) in ether (10 ml) gave E-tricarbonyl-(2,3,4-η-3-nonen-2-yl)-1-benzylaminocarbonyl iron (4) (500 mg, 82%) m.p. 80—86°, $\nu_{max.}$ 2900, 2850, 2030, 1995, 1980 and 1590 $cm^{-1}$; δ 7.31—7.10 (5H, m), 4.64 (1H, dd, J 12 and 8 Hz), 4.31 (1H, ABq, J 14 Hz), 4.17 (1H, m), 4.16 (1H, ABq, J 14 Hz); 3.78 (1H, m), 3.26 (1H, ABX, J 11 and 6 Hz), 3.10 (1H, ABX, J 11 Hz), 2.3402.24 (1H, m), 1.79—1.68 (1H, m), 1.65—1.41 (2H, m), 1.35 (4H, bd, s), and 0.91 (3H, t, J 6 Hz), (Found: C, 60.5; H, 5.80; N, 3.50. $C_{20}H_{23}NO_4Fe$ requires, C, 60.6; H, 5.90; N, 3.5%).

Description 5
Tricarbonyl-(1,1′,2-η-ethylcyclopent-1-ene-1′-yl)-2′-benzylaminocarbonyl iron (5)

A mixture of syn and anti tricarbonyl (1,1′,2′-η-1-vinyl-cyclopentan-1-yl)-2-oxycarbonyl iron (1 g, 3.6 mmol) with zinc chloride (1 g, 7.35 mmol) and benzylamine (5.0 g, 46.7 mmol) in ether (70 ml) gave tricarbonyl-(1,1′,2-η-ethylcyclopent-1-en-1′-yl)-2′-benzylaminocarbonyl iron (5) (0.5 g, 38%) (48% based on starting material) m.p. 98—100° dec., $\nu_{max.}$ 2900, 2020, 1990, 1980, 1580 and 680 $cm^{-1}$; δ 7.39—7.09 (5H, m), 4.30—4.06 (4H, m), 3.27 (2H, m), 2.60—2.26 (2H, m), and 2.14—1.60 (4H, m) (Found: C, 58.60; H, 4.55; N, 3.75 $C_{18}H_{17}NO_4Fe$ requires C, 58.88; H, 4.66; N, 3.80%).

Description 6
Tricarbonyl (1,1′,2′-η-1-vinyl-cyclopentan-1-yl)-2-benzylaminocarbonyl iron (6a) and tricarbonyl (1,1′-2-η-ethylcyclopent-1-ene-1′-yl)-2′-benzylaminocarbonyl iron (6b)

Tricarbonyl-(1,1′,2-η-ethylcyclopent-1-en-1-yl)-2′-oxycarbonyl iron (100 mg, 0.36 mmol) with zinc chloride (98 mg, 0.72 mmol) and benzylamine (385 mg, 3.6 mmol) in THF/ether (1:4, 10 ml) gave tricarbonyl (1,1′,2′-η-1-vinyl-cyclopentan-1-yl)-2-benzylaminocarbonyl iron (6a) (52 mg, 39%) m.p. 95—98°, $\nu_{max.}$ 3020, 2960, 2860, 2070, 2010, 1960, 1590, 1170, 700, and 665 $cm^{-1}$; δ 7.34—7.18 (3H, m), 7.17—7.04 (2H, m), 5.07 (1H, dd, J 8 and 12 Hz), 4.88 (1H, ABq, J 14 Hz), 3.68 (1H, ABq, J 14 Hz), 3.44—3.32 (3H, m), and 2.65—1.52 (6H, m) (Found: C, 58.92; H, 4.66; N, 3.80. $C_{18}H_{17}NO_4Fe$ requires C, 58.88; H, 4.66; N, 3.80%) and tricarbonyl-(1,1′-2-η-ethylcyclopent-1-ene-1′-yl)-2′-benzylaminocarbonyl iron (6b) (9 mg, 7%) m.p. 95—8°, $\nu_{max.}$ 2900, 2020, 1990, 1980, 1580 and 680 $cm^{-1}$; δ 7.39—7.18 (3H, m), 7.16—7.07 (2H, m), 4.35 (1H ABq, J 15 Hz), 4.27 (1H, d, J 6 Hz), 4.22 (1H, bd, s), 4.13 (1H, ABz, J 15 Hz), 3.37 (1H, ABX, J 13 and 6 Hz), 3.21 (1H, ABq, J 13 Hz), 2.92—2.75 (1H, m), 2.48—2.37 (2H, m), 2.02 (1H, dd, J 15 and 8 Hz), 1.96—1.82 (1H, m), and 1.58—1.37 (1H, m) as an inseparable mixture.

Description 7
Tricarbonyl-(2,3,4-η-3-buten-2-yl)1-(carbomethoxy-p-benzyloxyphenyl)methylaminocarbonyl iron (7)

Tricarbonyl-(2,3,4-η-3-buten-zyl)-1-oxycarbonyl iron (38 mg, 0.16 mmol) with zinc chloride (43 mg, 0.3 mmol) and methyl-p-benzyloxyphenyl-C-glycinate (0.6 g, 2.2 mmol) in THF/ether (1:4, 13 ml) gave tricarbonyl-(2,3,4-η-3-buten-2-yl)-1-(carbomethoxy-p-benzyloxyphenyl)methylaminocarbonyl iron (7) (61 mg, 80%) as an oil, $\nu_{max.}$ 3060, 3030, 2950, 2870, 2090, 2010, 2000, 1740, 1610, 1590, 1510, 1240, 1160, 690 and 665 $cm^{-1}$.

Description 8

Tricarbonyl-(2,3,4-$\eta$-3-methyl-3-buten-2-yl)-1-p-nitrobenzylaminocarbonyl iron (8)

Tricarbonyl-(2,3,4-$\eta$-3-methyl-3-buten-2-yl)-1-oxycarbonyl iron (70 mg, 0.3 mmol) with zinc chloride (76 mg, 0.6 mmol) and p-nitrobenzylamine (45.6 mg, 3 mmol) in THF/ether (2:1, 9 ml) gave tricarbonyl-(2,3,4-$\eta$-3-methyl-3-buten-2-yl)-1-p-nitrobenzylaminocarbonyl iron (8) (89 mg, 85%), m.p. 118—123°, $\nu_{max.}$ 3040, 3000, 2950, 2870, 2095, 2025, 2000, 1580, 1525, 1355, 1215 and 750 $cm^{-1}$; $\delta$ 8.20 (2H, ABq, 5.8 Hz), 7.25 (2H, ABq, J 8 Hz), 4.39 (1H, ABq, J 15 Hz), 4.20 (1H, ABq, J 15 Hz), 3.68—3.51 (1H, m), 3.34—3.18 (2H, m), 2.66 (1H, bd, s) and 2.03 (3H, s) (Found: C, 50.02; H, 3.74; N, 7.25. $C_{16}H_{14}N_2O_6Fe$ requires C, 49.77; H. 3.65; N, 7.26%).

Description 9

Tricarbonyl-(2,3,4-$\eta$-3-methyl-3-buten-2-yl)-1-2,4-dimethoxy benzylaminocarbonyl iron (9)

Tricarbonyl-(2,3,4-$\eta$-3-methyl-3-buten-2-yl)-1-oxycarbonyl iron (150 mg, 0.6 mmol) with zinc chloride (81 mg, 0.6 mmol and 2,4-dimethoxybenzylamine (500 mg, 3 mmol) in ether (5 ml) gave tricarbonyl-(2,3,4-$\eta$-3-methyl-3-buten-2-yl)-1-2,4-dimethoxy benzylaminocarbonyl iron (9) (226 mg, 95%) m.p. 95—8° $\nu_{max.}$ 3000, 2955, 2945, 2060, 2000, 1960, 1610, 1585, 1505, 1210, 1035, 910 and 740 $cm^{-1}$ $\delta$ 7.06—6.94 (1H, m), 6.50—6.32 (2H, m), 4.16 (3H, m), 3.80 (6H, s), 3.56 (1H, m), 3.31—3.10 (2H, m), 2.73 (1H, bd, s) and 1.98 (3H, s) (Found: C, 54.01; H, 4.81; N, 3.51. $C_{18}H_{19}NO_6Fe$ requires C, 53.89; H, 4.77; N, 3.49%).

Description 10

Tricarbonyl-(2,3,4-$\eta$-3-methyl-3-buten-2-yl)-1-carbobenzoxy-p-benzyloxyphenyl)methylaminocarbonyl iron (10)

Tricarbonyl-(2,3,4-$\eta$-3-methyl-3-buten-2-yl)-1-oxycarbonyl iron (100 mg, 0.4 mmol) with zinc chloride (108 mg, 0.8 mmol) and benzyl-p-benzyloxyphenyl-c-glymate (1.4 g, 4 mmol) in THF/ether (1:1, 10 ml) gave tricarbonyl-(2,3,4- -3-methyl-3-buten-2-yl)-1-carbobenzoxy-p-benzyloxyphenyl)-methylaminocarbonyl iron (10) (103 mg, 45%) as an oil, $\nu_{max}$ 3090, 3070, 3040, 2960, 2930, 2070, 2020, 1980, 1740, 1610, 1588, 1510, 1250 and 1170 $cm^{-1}$.

*General Procedure for the Oxidation of Tricarbonyl iron Lactam Complexes*

Ceric ammonium nitrate in ethanol was added to a solution of the tricarbonyl iron lactam complex in ethanol at low temperature. The mixture was warmed to room temperature slowly and stirred until the reaction was complete (as indicated by thin layer chromatography). The solvent was removed under reduced pressure and water added. The aqueous layer was extracted with ether, the combined ethereal extracts dried and the solvent removed under reduced pressure to give the crude product, which was purified by chromatography.

Example 1

1-Benzyl-3-ethenyl-3-methyl-azetidinone

(1) (110 mg, 0.34 mmol) with ceric ammonium nitrate (820 mg, 1.5 mmol) in ethanol (10 ml) at −30°C gave 1-benzyl-3-ethenyl-3-methyl-azetidinone (45 mg, 72%) as an oil; $\nu_{max.}$ 3085, 3030, 3000, 2980, 2965, 2920, 2900, 1745, 1645, 1400, 940, and 700 $cm^{-1}$; $\delta$ 7.39—7.21 (5H, m), 6.12—5.78 (1H, m), 5.42—5.16 (2H, m), 4.40 (2H, s), 3.98—3.76 (1H, m), 3.39 (1H, dd, J 5 and 5 Hz), and 3.06 (1H, dd, J 5 and 3 Hz) (Found: $M^+$ 187.0999, $C_{12}H_{13}NO$ requires M 187.0997).

Example 2

1-benzyl-3-isoprenyl azetidinone

(2) (90 mg, 0.26 mmol) with ceric ammonium nitrate (0.9 g, 1.64 mmol) in ethanol (10 ml) at −30° gave 1-benzyl-3-isoprenyl-azetidinone (40 mg, 75%) as an oil, $\nu_{max.}$ 3080, 3060, 3030, 2970, 2900, 1745, 1645, 1605, 1400, 900, 730 and 700 $cm^{-1}$; $\delta$ 7.36—7.17 (5H, m), 4.92 (2H, bd, s), 4.38 (2H, s), 3.90—3.72 (m, 1H), 3.26 (1H, dd, J 6 and 6 Hz), 3.04 (1H, dd, J 6 and 3 Hz), and 1.78 (3H, s) (Found: $M^+$ 201.1153, $C_{13}H_{15}NO$ requires M 201.1154).

Example 3

1-benzyl-3-isoprenyl-3-methyl-azetidinone

(3) (0.32 g, 0.9 mmol) with ceric ammonium nitrate (2.28 g, 4.00 mmol) in ethanol (10 ml) at 0°C gave 1-benzyl-3-isoprenyl-3-methyl-azetidinone (65 mg, 33.5%) as an oil, $\nu_{max.}$ 2860, 1740, 1640, 1400 and 700 $cm^{-1}$; $\delta$ 7.37—7.17 (5M, m), 5.02 (1H, bd, s), 4.86 (1H, m), 4.40 (2H, s), 3.16 (1H, ABq, J 5 Hz), 2.90 (1H, ABq, J 5 Hz), 1.77 (3H, s) and 1.45 (3H, s) (Found: $M^+$ 215.1310. $C_{14}H_{17}NO$ requires M 215.1309) and 1-benzyl-3,6-dihydro-4,5-dimethyl-2-pyridone (20) (109 mg, 56.3%) m.p. 78—80°, $\nu_{max.}$ 2900, 1640, 1495, 1450, 1320, 1265, 725 and 715 $cm^{-1}$; $\delta$ 7.35—7.18 (5H, m), 4.73 (2H, s), 3.73 (2H, bd, s), 3.03 (2H, bd, s) and 1.70 (6H, s) (Found: C, 78.00; H, 8.15; N, 6.35. $C_{14}H_{17}NO$ requires C, 78.15; H, 7.90; N, 6.50%).

Example 4

1-benzyl-3-(1-heptenyl)-azetidinone

(4) (0.63 g, 1.58 mmol) with ceric ammonium nitrate (4.1 g, 7.5 mmol) in ethanol (10 ml) at −5° give cis- and trans-1-benzyl-3-(1-heptenyl)-azetidinone (0.26 g, 64%) as an oil, $\nu_{max.}$ 2900, 1745, and 1390 $cm^{-1}$; $\delta$ 7.36—7.20 (5H, m) 6.00—5.24 (2H, m), 4.38 (2H, s), 3.88—3.66 (1H, m) 3.32 (1H, dd, J 6 and 6 Hz), 2.93 (1H, dd, J 6 and 2.5 Hz), 2.28—1.90 (2H, m), 1.68—1.04 (6H, m), and 1.00—0.72 (3H, m) (Found: $M^+$ 257.1772, $C_{17}H_{23}NO$ requires M 257.1779).

Example 5

1-benzyl-3-(cyclopenten-1′-yl)-azetidinone

(5) (190 mg, 0.517 mmol) with ceric ammonium nitrate (1.35 g, 2.46 mmol) in ethanol (23 ml) at ambient temperature gave 1-benzyl-3-(cyclopenten-1′-yl)-azetidinone (99 mg, 84%) as an oil, $\nu_{max.}$ 2860 and 1740 $cm^{-1}$; $\delta$ 7.38—7.20 (5H, m), 5.66 (1H, bd, s), 4.45 (2H, s), 4.10—3.86 (1H, m), 3.34 (1H, dd, J 5 and 5 Hz), 3.08 (1H, dd J 5 and 3 Hz), and 2.56—1.84 (6H, m) (Found: C, 79.00; H, 7.70; N, 6.20. $C_{15}H_{17}NO$ requires C, 79.25; H, 7.55; N, 6.15%).

Example 6

1-vinyl-(N-benzyl)-6-azabicyclo [3.2.0]hept-7-one

A mixture of (6a) and (6b) (80 mg, 0.2 mmol) with ceric ammonium nitrate (0.5 g, 0.91 mmol) in ethanol (15 ml) at −30° gave 1-vinyl-(N-benzyl)-6-azabicyclo [3.2.0]heptan-7-one (37 mg) 75%) as an oil, $\nu_{max.}$ 2950, 2900, 1740, 1635, 1300 and 650 $cm^{-1}$; $\delta$ 7.42—7.16 (5H, m), 6.18—5.90 (1H, m), 5.46—5.08 (2H, m), 4.49 (1H, ABq, J 15 Hz), 4.22 (1H, ABq, J 15 Hz), 3.71 (1H, d, J 4 Hz) and 2.24—1.12 (6H, m) (Found: $M^+$ 227.1312. $C_{15}H_{17}NO$ requires M 227.1310.

Example 7

1-(carbomethoxy-p-benzylphenyl)methyl-3-ethyl-3-ethynyl-azetidinone

(7) (61 mg, 0.12 mmol) with ceric ammonium nitrate (0.5 g, 0.9 mmol) in ethanol (23 ml) at −30° gave 1-carbomethoxy-p-benzyloxyphenyl)methyl-3-ethyl-3-ethynyl-azetidinone (35 mg, 81%) as an oil, $\nu_{max.}$ 3090, 3060, 3030, 3000, 2980, 2955, 2900, 1755, 1740, 1640, 1610, 1585, 1510, 1230, 1180, 735 and 695 $cm^{-1}$; $\delta$ 7.40—7.26 (5H, m), 7.24 (2H, ABq, J 8 Hz), 7.00 (2H, ABq, J 8 Hz), 6.18—5.80 (1H, m), 5.60 (1H, s), 5.44—5.20 (2H, m), 5.08 (2H, s), 3.92—3.70 (4H, m), 3.56—3.42 (1H, m), and 3.38—3.28 (1H, m) (Found: $M^+$ 351.1468. $C_{21}H_{21}NO_4$ requires M 351.1470).

Example 8

1-p-nitrobenzyl-3-isopropenylazetidinone

(8) (150 mg, 0.4 mmol) with ceric ammonium nitrate (853 g, 1.6 mmol) in ethanol (20 ml) at −30° gave 1-p-nitrobenzyl-3-isopropenylazetidinene (70 mg, 74%) as an oil; $\nu_{max.}$ 3080, 2950, 2915, 2850, 1740, 1640, 1600, 1520, 1340 and 1100 $cm^{-1}$; 8.20 (2H, ABq, 9 Hz), 7.42 (2H, ABq, 9 Hz), 4.96 (2H, m), 4.59 (1H, ABq, J 16 Hz), 4.41 (1H, ABq, J 16 Hz), 3.89 (1H, m), 3.36 (1H, dd, J 5.6 and 5.5 Hz), 3.10 (1H, dd, J 5.6 and 2.8 Hz), and 1.78 (3H, s) (Found: $M^+$ 246.1007. $C_{13}H_{14}N_2O_3$ requires 246.1004).

Example 9

1-2,4-dimethoxybenzy-3-isopropenyl azetidinone

(9) (226 mg, 0.56 mmol) with ceric ammonium nitrate (1.24 g 2.2 mmol) in ethanol (15 ml) at −30° gave 1-2,4-dimethoxybenzyl-3-isopropenyl azetidinone (8 mg, 13%) as an oil; $\nu_{max.}$ 3075, 2950,, 2925, 1740, 1640, 1405, 1500, 1200, 1030 and 820 $cm^{-1}$; $\delta$ 7.16—6.97 (1H, m), 6.52—6.30 (2H, m), 4.97—4.82 (2H, m), 4.38 (1H, ABq, J 14 Hz), 4.25 (1H, ABq, J 14 Hz), 3.90—3.66 (7H, m), 3.22 (1H, dd, J 5 and 5 Hz), 2.97 (1H, dd, J 5 and 3 Hz).

Example 10

1-(carbobenzoxy-p-benzyloxyphenyl)methyl-3-isopropenylazetidone

(10) (100 mg, 0.17 mmol) with ceric ammonium nitrate (0.4 g, 0.7 mmol) in ethanol (10 ml) at −30° gave 1-(carbobenzoxy-p-benzyloxyphenyl)methyl-3-isopropenylazetidinone (36) (46 mg, 60%) as a diastereomeric mixture of oils. $\nu_{max.}$ 2970, 1750, 1740, 1645, 1610, 1370 and 695 $cm^{-1}$; $\delta$ 7.26 (5H, s), 7.17 (5H, s), 7.10—6.70 (4H, m), 5.50 (1H, s), 5.15 (2H, s), 5.01 (2H, s), 4.98—4.84 (2H, bd, s), 3.79—2.90 (3H, m) and 1.60 (3H, s).

*General method for Ozonolysis of β-lactams*

A solution of the β-lactam in dichloromethane at −178° was exposed to ozone until an excess of ozone was seen (as indicated by the blue colour of the solution). The excess ozone was removed with argon and triphenyl phosphine added. The solution was warmed to room temperature, the solvent removed under reduced pressure and the crude product chromatographed.

Example 11

1-p-nitrobenzylamino-3-acetyl azetidinone

1-p-nitrobenzylamino-3-isopropenylazetidinone (40 mg, 0.16 mmol) in dichloromethane (10 ml) at —78° with ozone gave 1-p-nitrobenzyl-3-acetyl azetidinone (32 mg, 80%) as an oil, $\nu_{max.}$ 1760, 1720, 1615, 1525 and 1355 $cm^{-1}$; $\delta$ 8.20 (2H, ABq, J 9 Hz), 7.36 (2H, ABq, J 9 Hz), 4.56 (2H, s), 4.29 (1H, dd, J 3 and 5 Hz), 3.64 (1H, dd, J 5 and 3 Hz), 3.19 (1H, dd, J 5 and 5 Hz), and 2.33 (3H, s) (Found: $M^+$ 248.0792. $C_{12}H_{12}N_2O_4$ requries M 248.0797).

Example 12

1-(carbobenzoxy-p-benzyloxyphenyl)-methyl-3-isopropenyl azetidinone

1-(Carbobenzoxy-p-benzyloxyphenyl)methyl-3-acetyl azetidinone (150 mg, 0.34 mmol) in methylene chloride (15 ml) at —78° with ozone gave 1-(carbobenzoxy-p-benzyloxyphenyl)-methyl-3-acetyl azetidinone (111 mg, 74%) as a mixture of diastereomeric oils, $\nu_{max.}$ 1760, 1740, 1720, 1610, 1510, 1250 and 695 $cm^{-1}$; $\delta$ 7.47—6.80 (14H, m), 5.48 (1H, s), 5.14 (2H, s), 5.00 (2H, s), 4.24—3.83 (2H, m), 3.67—3.32 (1H, m) and 2.30 (3H, s).

Example 13

1-benzyl-3-1′,2′-epoxyethyl-azetidinone

1-Benzyl-3-ethenyl-3-methyl-azetidinone (205 mg, 1.1 mmol) in dichloromethane (10 ml) with sodium carbonate (200 mg, 2 mmol) and m-chloroperbenzoic acid (260 mg, 80%, 1.2 mmol) was stirred for 24 h. The suspension was filtered, washed with aqueous sodium bicarbonate solution (5 ml) and the organic layers dried ($MgSO_4$). Removal of the solvent under reduced pressure and chromatography of the resulting oil (silica, ethyl acetate/petroleum ether (60/80) gave 1-benzyl-3-1′,2′-epoxyethyl-azetidinone (80 mg, 36%) (50% based on recovered starting material) as an oil, $\nu_{max}$ 3060, 2980, 1750, 1600, 1395 and 700 $cm^{-1}$; $\delta$ 7.52—7.15 (5H, m), 4.36 (2H, s), 3.7—3.50 (1H, m) and 3.33—2.46 (5H, m).

Example 14

1-p-nitrobenzylamino-3-1′-hydroxyethyl azetidinone (32)

1-p-nitrobenzylamino-3-acetyl azetidinone (70 mg, 0.3 mmol) with sodium borohydride (5 mg, 0.15 mmol) in ethanol at 0° was stirred for 1 h. Removal of the solvent and chromatography of the resulting oil (silica ethyl acetate) gave 1-p-nitrobenzylamino-3-1′-hydroxyethyl azetidinone (50 mg, 71%) as an oil, $\nu_{max.}$ 3450, 3060, 2940, 2910, 1740, 1610, 1525, 1350, 1050, and 640 $cm^{-1}$; $\delta$ 8.16 (2H, ABq, J 8 Hz), 7.47 (2H, ABq, J 8 Hz), 4.57 (1H, ABq, J 16 Hz), 4.36 (1H, ABq, J 16 Hz), 4.30—3.90 (1H, m), 3.40—2.78 (4H, bd, m) and 1.32 (3H, d, J 9 Hz).

Example 15

1-benzyl-3-1′-hydroxyethyl azetidinone (34)

1-Benzyl-3-1′,2′-epoxyethyl-azetidinone (55 mg, 0.3 mmol) with K-electrode (0.6 ml, solution in THF, 0.3 mmol) in THF (5 ml) at —78° was warmed to room temperature and stirred for 6 h. The reaction mixture was washed with water (40 ml), extracted with ethyl acetate (3 x 40 ml) and the combined organic layers dried ($MgSO_4$). Removal of the solvent under reduced pressure and chromatographed on the resulting oil gave 1-benzyl-3-1′-hydroxyethyl azetidinone (22 mg, 40%) as an oil, $\nu_{max.}$ 3240, 3060, 2990, 1740, 1440, 1265 and 705 $cm^{-1}$; $\delta$ 7.30 (5H, m,), 4.36—4.00 (3H, m), 3.31—2.88 (3H, m) and 1.25 (3H, d, J 6 Hz).

**Claims**

1. A process for the preparation of a lactam of formula (II):

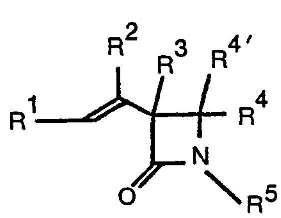

(II)

wherein $R^1$, $R^2$ and $R^3$ may be the same or different and each represents hydrogen or $C_{1-6}$ alkyl, or $R^1$ and $R^2$ may together complete a carbocyclic ring; $R^4$ is hydrogen, $—CH_2—CO_2R^p$ where $R^p$ is a carboxy-protecting group, $—CH_2—CH_2—OH$ or a protected derivative thereof, $—CH_2—CH_2—CHO$ or a protected derivative thereof, or $R^3$ and $R^4$ together complete a carbocyclic ring; $R^{4'}$ is hydrogen or $C_{1-6}$ alkyl; and $R^5$ is hydrogen, an organic radical wherein any reactive groups may be protected or a subgroup of formula-$NR^\alpha R^\beta$ wherein $R^\alpha$ and $R^\beta$ are the same or different and each is hydrogen or an

organic radical, or a subgroup of $OR^\delta$ wherein $R^\delta$ is a hydroxyl protecting group, which process comprises treatment of an iron complex of formula (III):

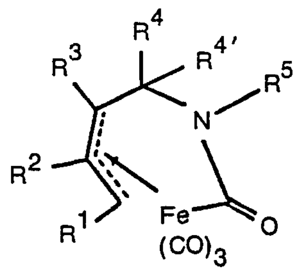

(III)

wherein $R^1$, $R^2$, $R^3$, $R^4$, $R^{4'}$ and $R^5$ are as defined with respect to a formula (II), with an oxidising agent.

2. A process as claimed in claim 1 wherein the oxidising agent is an iron III salt, silver I salt or a cerium IV salt.

3. A process as claimed in claim 1 or claim 2 wherein the oxidising agent is a cerium IV salt.

4. A process as claimed in any one of claims 1 to 3 wherein the oxidising agent is cerium IV ammonium nitrate.

5. A process as claimed in any one of claims 1 to 4 wherein $R^5$ is hydrogen, an amino protecting group, or a sub-group of formula:

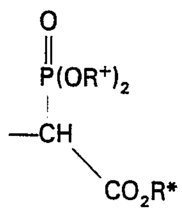

wherein $R^+$ is $C_{1-6}$ alkyl and $R^*$ is an organic group such that $—CO_2R^*$ is an ester group.

**Revendications**

1. Procédé pour la préparation d'un lactame de formule (II):

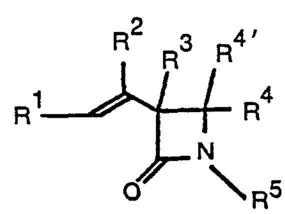

(II)

dans laquelle $R^1$, $R^2$ et $R^3$ peuvent être identiques ou différents et représentent chacun de l'hydrogène ou un alcoyle en $C_{1-6}$, ou bien $R^1$ et $R^2$ peuvent former ensemble un carbocycle; $R^4$ est de l'hydrogène, $—CH_2—CO_2R^p$ où $R^p$ est un groupe de protection du carboxyle, $—CH_2—CH_2—OH$ ou un dérivé protégé de celui-ci, $—CH_2—CH_2—CHO$ ou un dérivé protégé de celui-ci, ou bien $R^3$ et $R^4$ forment ensemble un carbocycle; $R^{4'}$ est de l'hydrogène ou un alcoyle en $C_{1-6}$; et $R^5$ est de l'hydrogène, un radical organique dans lequel des groupes réactifs quelconques peuvent être protégés ou un sous-groupe de formule $—NR^\alpha R^\beta$, où $R^\alpha$ et $R^\beta$ sont identiques ou différents et désignent chacun de l'hydrogène ou un radical organique, ou bien un sous-groupe de formule $OR^\delta$ où $R^\delta$ est un groupe de proteciton de l'hydroxy, ce procédé consistant à effectuer le traitement d'un complexe de fer de formule (III):

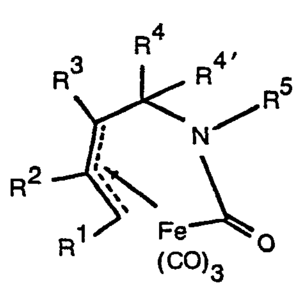

(III)

dans laquelle $R^1$, $R^2$, $R^3$, $R^4$, $R^{4'}$ et $R^5$ sont tels que définis dans le cas de la formule (II), avec un agent d'oxydation.

2. Procédé suivant la revendication 1, caractérisé en ce que l'agent d'oxydation est un sel de fer —III un sel d'argent —I ou un sel de cérium —IV.

3. Procédé suivant la revendication 1 ou 2, caractérisé en ce que l'agent d'oxydation est un sel de cérium —IV.

4. Procédé suivant l'une quelconque des revendications 1 à 3, caractérisé en ce que l'agent d'oxydation est de l'ammononitrate de cérium —IV.

5. Procédé suivant l'une quelconque des revendications 1 à 4, caractérisé en ce que $R^5$ est de l'hydrogène, un groupe de protection de l'amino ou un sous-groupe de formule:

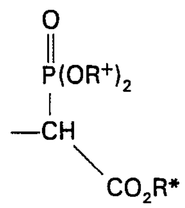

dans laquelle $R^+$ est un alcoyle en $C_{1-6}$ et $R^*$ est un groupe organique tel que $—CO_2R^*$ soit un groupe ester.

**Patentansprüche**

1. Ein Verfahren zur Herstellung eines Lactams der Formel (II)

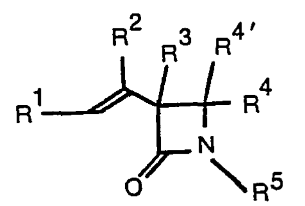

(II)

in welcher $R^1$, $R^2$ und $R^3$ gleich oder verschieden sein kön- und jeweils Wasserstoff oder $C_{1-6}$-Alkyl bedeuten oder $R^1$ und $R^2$ zusammen einen carbocyclischen Ring vervollständigen, $R^4$ Wasserstoff, eine Gruppe $—CH_2—CO_2R^p$, in welcher $R^p$ eine Schutzgruppe für den Carboxylrest ist, eine $—CH_2—CH_2—OH$-Gruppe oder ein geschütztes Derivat derselben, eine Gruppe $—CH_2CH_2—CHO$ oder ein geschütztes Derivat derselben bedeutet, oder $R^3$ und $R^4$ zusammen einen carbocyclischen Ring vervollständigen, $R^{4'}$ Wasserstoff oder $C_{1-6}$-Alkyl ist und $R^5$ Wasserstoff, ein organischer Rest, in welchem möglicherweise darin vorhandene reaktive Gruppen geschützt sind, oder eine Untergruppe der Formel $—NR^\alpha R^\beta$, in welcher $R^\alpha$ und $R^\beta$ gleich oder verschieden sind und jeweils Wasserstoff oder einen organischen Rest bedeuten, oder eine Untergruppe $OR^\delta$ ist, in welcher $R^\alpha$ eine die Hydroxylgruppe schützende Gruppe bedeutet, welches Verfahren die Behandlung eines Eisenkomplexes der Formel (III)

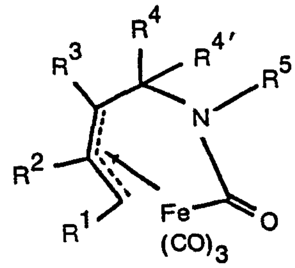

(III)

in welcher $R^1$, $R^2$, $R^3$, $R^4$, $R^{4'}$ und $R^5$ wie in bezug auf Formel (II) definiert sind, mit einem oxidierenden Mittel Mittel umfaßt.

2. Ein Verfahren wie in Anspruch 1 beansprucht, in welchem das oxidierende Mittel ein Eisen(III)-Salz, ein Silber-(I)-Salz oder ein Cer (IV)-Salz ist.

3. Ein Verfahren wie in Anspruch 1 oder 2 beansprucht, in welchem das oxidierende Mittel ein Cer (IV)-Salz ist.

4. Ein Verfahren wie in irgendeinem der Ansprüche 1 bis 3 beansprucht, in welchem das oxidierende Mittel Cer(IV)-Ammoniumnitrat ist.

5. Ein Verfahren wie in irgendeinem der Ansprüche 1 bis 4 beansprucht, in welchem $R^5$ Wasserstoff, eine Schutzgruppe für die Aminogruppe oder eine Untergruppe der Formel

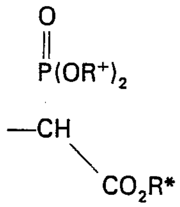

ist, in welcher $R^+$ $C_{1-6}$-Alkyl und $R^*$ eine organische Gruppe derart ist, daß $—CO_2R^*$ eine Estergruppe bedeutet.